# FASCICULE DE BREVET EUROPEEN

(11) **EP 1 111 383 B1**
(45) Date de publication et mention de la délivrance du brevet: **22.02.2006**
(21) Numéro de dépôt: 00403620.8
(22) Date de dépôt: 21.12.2000
(51) Int. Cl.: G01N 33/28, G01N 27/02, G01R 27/22

(54) **Capteur d'usure d'huile**
Ölalterungssensor
Oil degradation sensor

(30) Priorité: 24.12.1999 FR 9916466
(43) Date de publication de la demande: 27.06.2001
(73) Titulaire: SC2N, 78180 Montigny-le-Bretonneux (FR)
(72) Inventeur: Edet, Stéphanie, 14150 Ouistreham (FR)
(74) Mandataire: Texier, Christian

(56) Documents cités:
- WO-A-98/50790
- GB-A- 2 306 660
- US-A- 5 489 849
- US-A- 5 631 568
- FICHTNER W ET AL: "ON-LINE-MESSUNG DER EIGENSCHAFTEN VON SCHMIEROELEN FUER VERBRENNUNGSMOTOREN MIT EINEM ELEKTRISCHEN SENSOR" TECHNISCHES MESSEN TM,DE,R.OLDENBOURG VERLAG. MUNCHEN, vol. 65, no. 2, 1 février 1998 (1998-02-01), pages 53-57, XP000768823 ISSN: 0171-8096

## Description

La présente invention concerne le domaine des dispositifs de contrôle/mesure d'usure de lubrifiants, notamment d'huile utilisée sur véhicule automobile.

De nombreux dispositifs ont déjà été proposés, notamment à base de systèmes capacitifs, pour essayer de procéder à la mesure de l'usure d'huile ou lubrifiants équivalents.

Le document WO 98/52073 divulgue un dispositif d'analyse des fluides, notamment de lubrifiants par mesure de son impédance, notamment de la phase de son impédance.

Il propose en particulier de mesurer l'évolution de la phase de l'impédance en fonction de la fréquence d'excitation.

Cependant, les dispositifs jusqu'ici proposés s'avèrent à la fois complexes et peu précis.

En pratique, les usagers sont ainsi réduits de nos jours à opérer un contrôle régulier du niveau d'huile et à un ajout d'huile neuve si le niveau chute sous un seuil déterminé, avec vidanges régulières et complètes quant un kilométrage donné d'utilisation est atteint.

La présente invention a maintenant pour but de proposer un nouveau système simple, fiable et économique.

Ce but est atteint dans le cadre de la présente invention grâce à un dispositif de mesure de la phase tel que défini en revendication 1 annexée.

Selon une autre caractéristique avantageuse de l'invention, le dispositif procède à une surveillance de l'évolution de la phase de l'impédance et à une comparaison de la phase de l'impédance avec un seuil spécifique pour le déclenchement d'une alerte de "vidange ou maintenance" quand ce seuil spécifique est franchi par défaut par la phase de l'impédance.

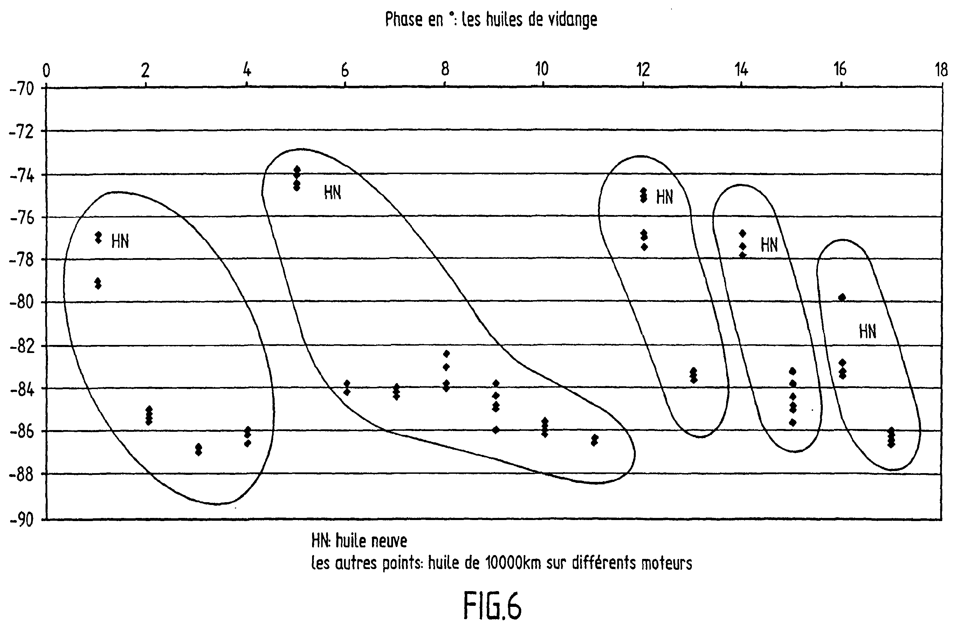

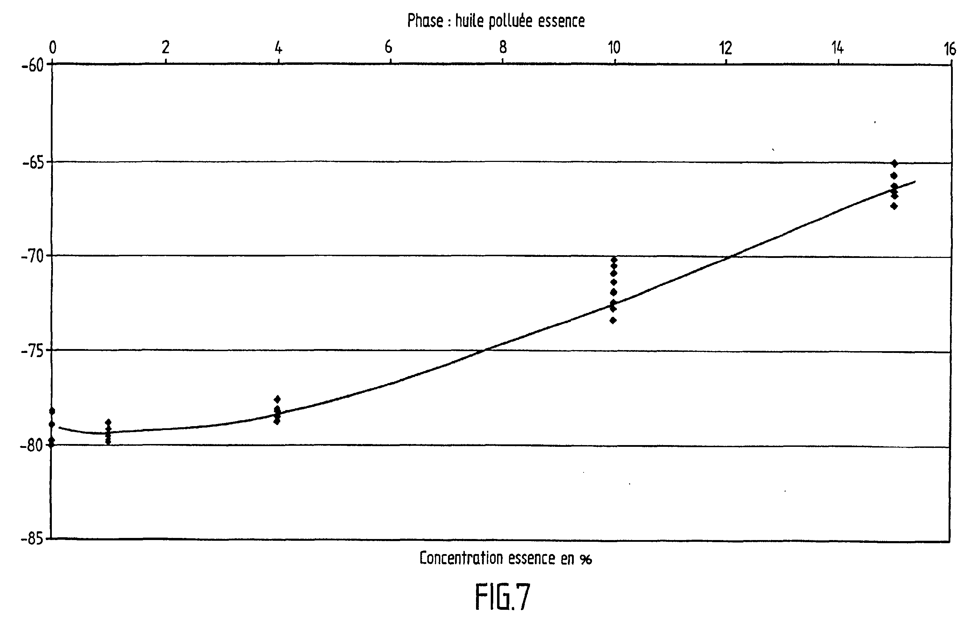

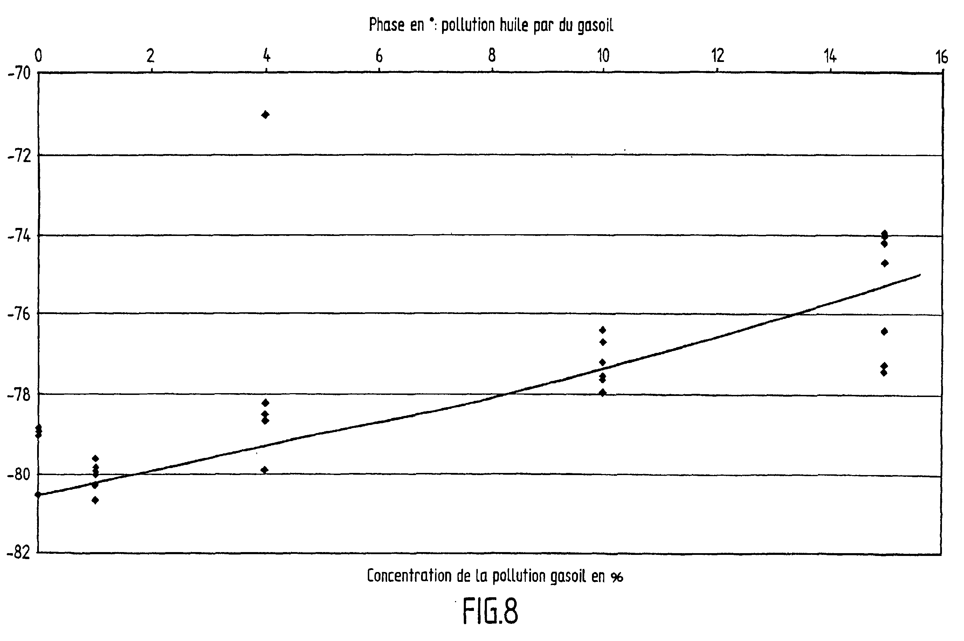

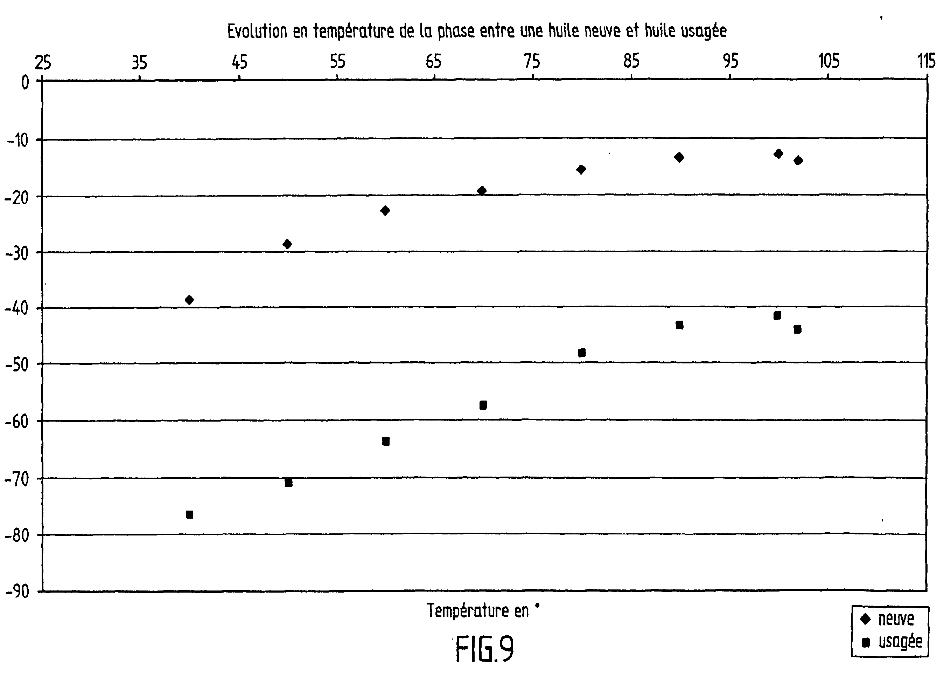

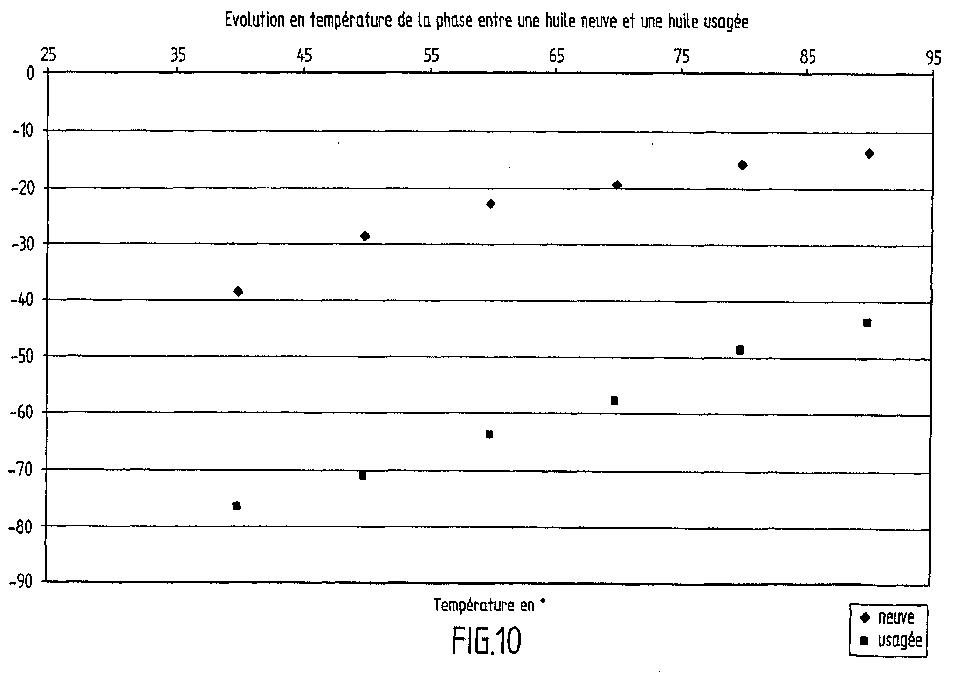

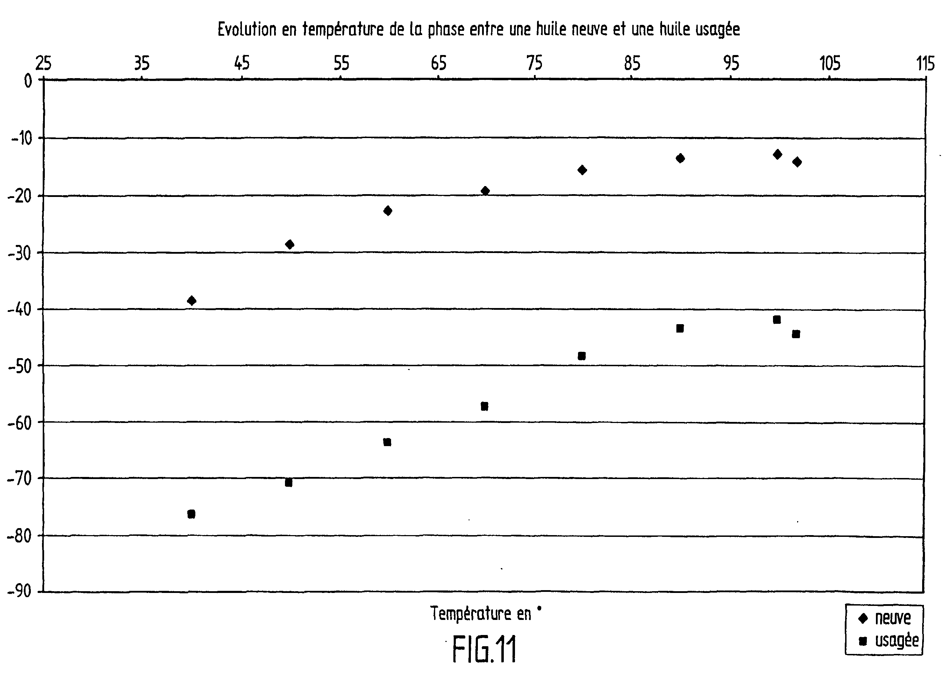

D'autres caractéristiques, buts et avantages de la présente invention apparaîtront à la lecture de la description détaillée qui va suivre et en regard des dessins annexés, donnés à titre d'exemple non limitatif et sur lesquels :
- la figure 1 représente une vue schématique d'un dispositif de mesure conforme à la présente invention,
- les figures 2 à 11 représentent des courbes expérimentales relevées sur différents échantillons d'huile et illustrent la phase de l'impédance de ces huiles en fonction des différents paramètres ; plus précisément :
   . la figure 2 représente l'évolution de la phase de l'impédance d'une huile pour moteur à essence en fonction du nombre d'heures d'utilisation sur un banc moteur,
   . la figure 3 représente la phase de l'impédance d'une huile pour moteur à essence en fonction du kilométrage,
   . la figure 4 représente la phase de l'impédance d'une huile pour moteur diesel en fonction du kilométrage,
   . la figure 5 représente la phase de l'impédance d'une huile pour moteur diesel en fonction du kilométrage véhicule exprimé en milliers de kilomètres,
   . la figure 6 représente la phase de l'impédance d'huiles de vidange pour différentes familles d'huiles dont les valeurs correspondant à des huiles neuves sont assorties de la référence HN tandis que les valeurs des huiles usagées correspondent à des huiles ayant été utilisées environ 10 000km sur différents moteurs,
   . la figure 7 représente la phase de l'impédance d'huiles polluées par de l'essence en fonction de la concentration de l'essence en %,
   . la figure 8 représente la phase de l'impédance d'huiles polluées par du gasoil en fonction de la concentration de la pollution gasoil en %,
   . la figure 9 représente l'évolution de la phase de l'impédance en fonction de la température pour une huile neuve et une huile usagée,
   . les figures 10 et 11 représentent des courbes similaires à la figure 9 pour d'autres types d'huile,
- la figure 12 représente schématiquement l'évolution de la phase de l'impédance d'une huile dans le temps, en fonction d'un usage normal, et
- la figure 13 représente l'évolution de la phase de l'impédance en cas de pollution accidentelle de l'huile, par du carburant ou un autre élément polluant tel que du liquide de refroidissement.

Le dispositif conforme à la présente invention comprend des moyens 10 conçus pour mesurer la phase de l'impédance d'un échantillon d'une huile à surveiller.

Ces moyens 10 comprennent de préférence des moyens aptes à appliquer une tension connue variable, par exemple sinusoïdale, entre deux électrodes 20, 22 électriquement conductrices, plongées dans l'échantillon d'huile considéré, et des moyens aptes à mesurer le déphasage entre la tension précitée et le courant résultant.

La phase de l'impédance dépend, bien entendu, de la valeur des composantes résistive, inductive et capacitive de l'huile, c'est-à-dire à la fois des éléments constitutifs fondamentaux de l'huile considérée, mais également des éléments susceptibles de provenir d'une usure ou d'une pollution de l'huile, tels que des résultats d'oxydation, d'une pollution par un carburant, essence ou diesel, un liquide de refroidissement, de l'eau, ou tout autre corps étranger équivalent.

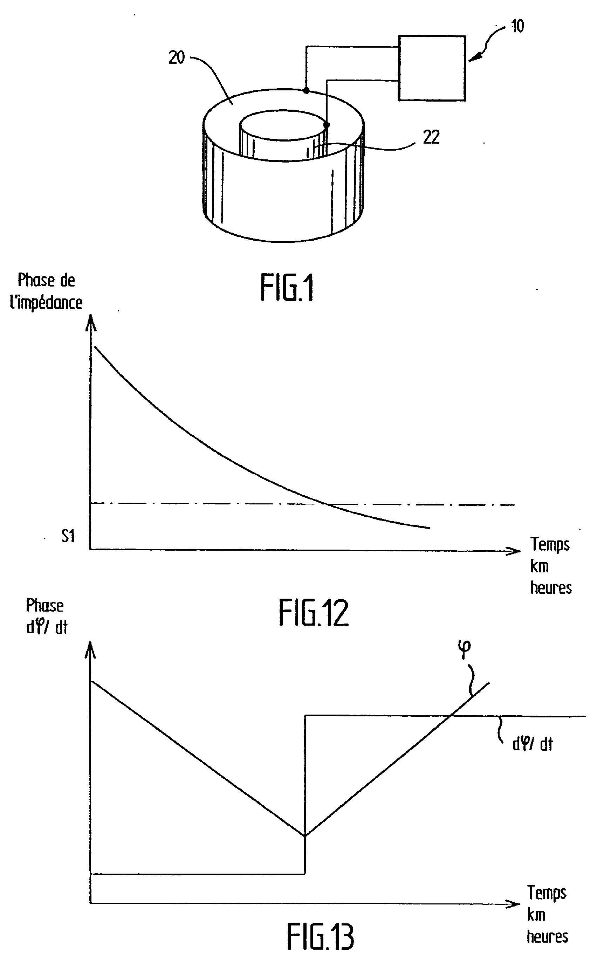

Selon le mode de réalisation schématisé sur la figure 1, les deux électrodes 20, 22 sont circulaires de révolution et concentriques.

Bien entendu l'invention n'est pas limitée à ce mode de réalisation précis.

A titre d'exemple non limitatif, les deux électrodes 20, 22 peuvent être formées de plaques parallèles.

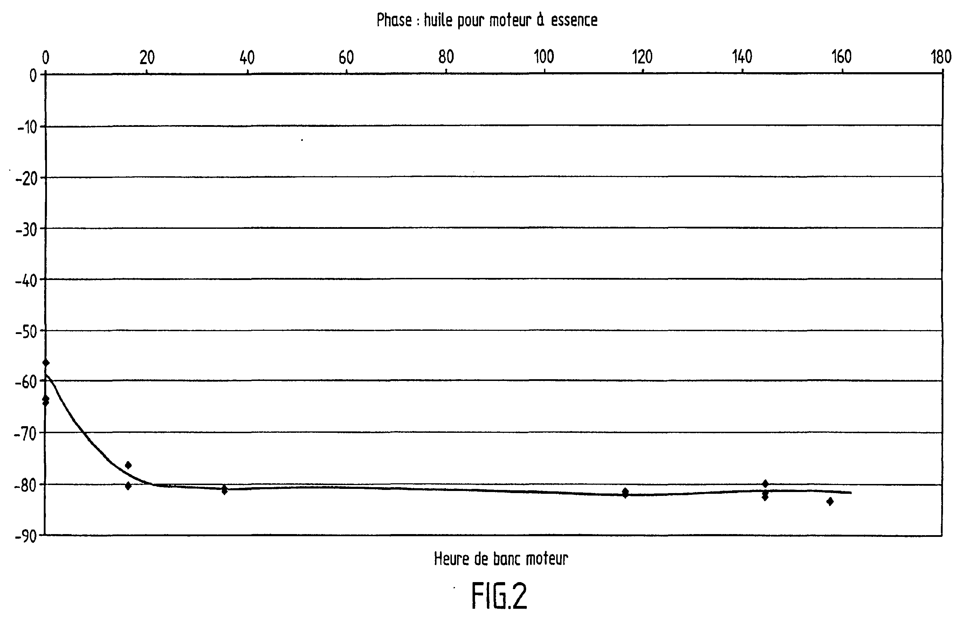

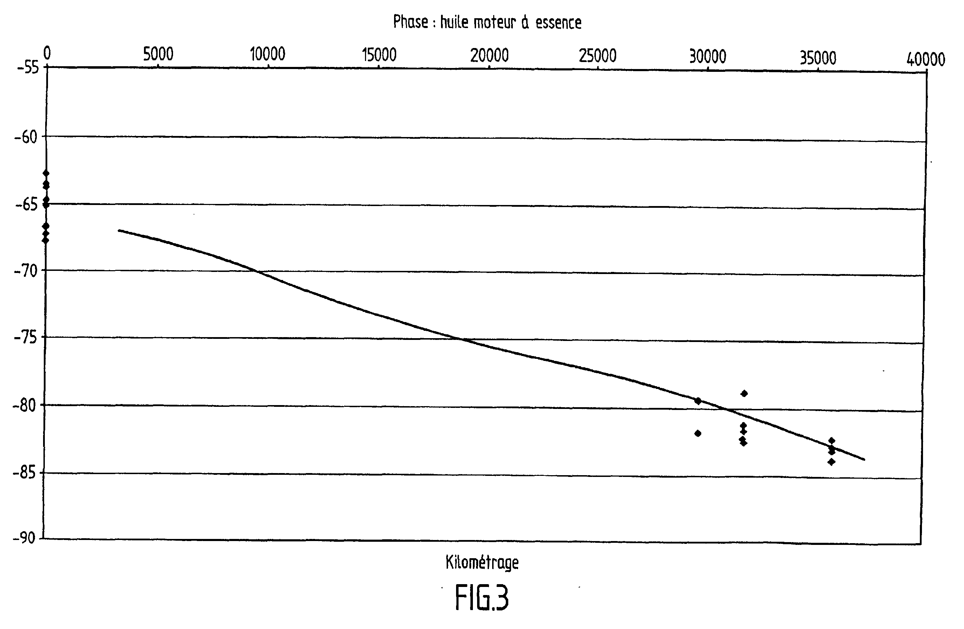

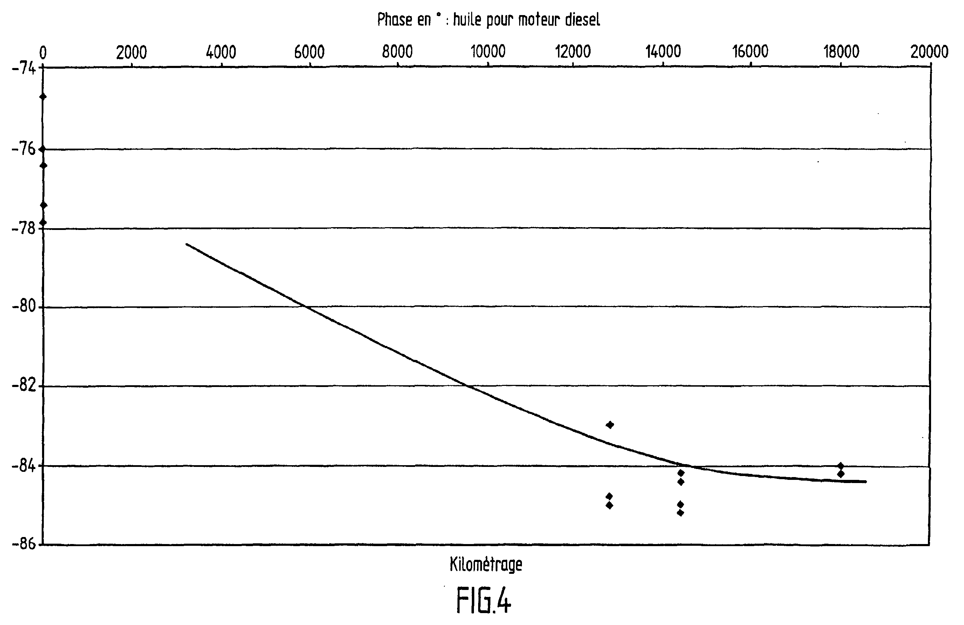

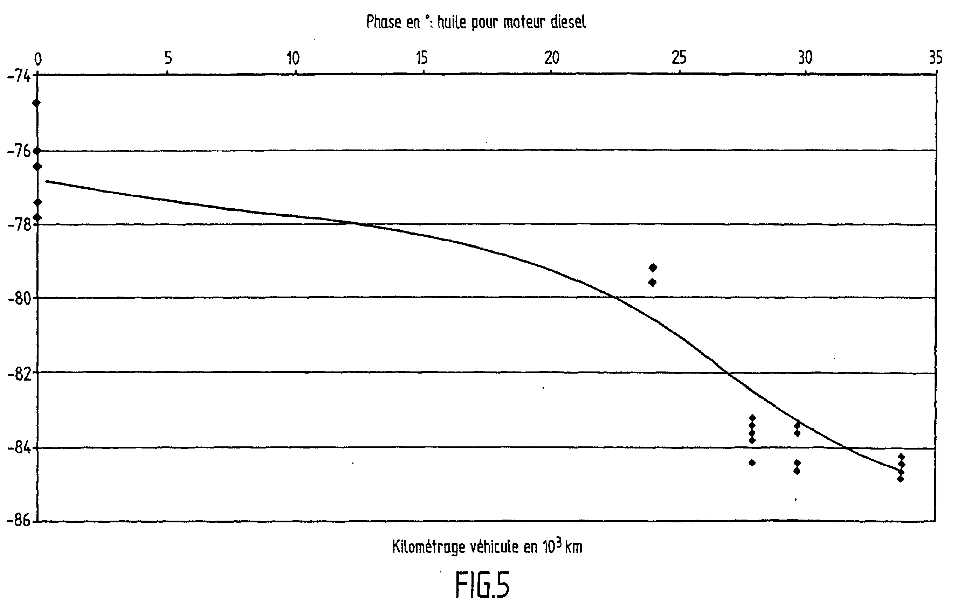

Les nombreux essais réalisés par la demanderesse, relatés en partie sur les figures 2 à 11 annexées, ont révélé un premier phénomène : l'oxydation de l'huile résultant de son usage normal conduit à une diminution progressive de la phase de l'impédance (voir figures 2 à 6).

Les figures 2 à 6 révèlent par exemple une évolution de la phase de l'impédance dans une gamme comprise entre environ -55° pour une huile neuve et environ -85° pour une huile usagée sur une gamme de fréquence.Bien entendu, comme on le voit sur les figures annexées, l'évolution de la phase de l'impédance peut ne pas couvrir intégralement cette gamme.

Ce phénomène est schématisé sur la figure 12 sur laquelle on a identifié par la référence S1, un seuil de phase d'impédance en deçà duquel on peut considérer que l'huile est hors d'usage.

Pour cela, de préférence, les moyens 10 conformes à la présente invention, comprennent des moyens aptes à comparer la phase de l'impédance de l'échantillon d'huile au seuil S1 et des moyens aptes à générer une alerte "de vidange ou maintenance" quand la phase de l'impédance franchit par défaut ce seuil S1. La génération de cette alarme signale à l'utilisateur la nécessité de remplacer l'huile par une huile neuve.

Les essais réalisés par la demanderesse ont révélé par ailleurs que l'amplitude de la phase de l'impédance de l'échantillon d'huile considéré peut varier d'une huile à l'autre. De ce fait, de préférence, la mesure est faite en différentiel ou en relatif et non point en valeur absolue. En d'autres termes, le seuil S1 utilisé correspond de préférence à un pourcentage de la valeur initiale mesurée pour la phase et non point à une valeur fixe et standard quelle que soit l'huile.

Les essais réalisés par la demanderesse ont révélé un second phénomène : en cas de pollution de l'huile par un corps étranger tel qu'un carburant du type essence ou diesel, voire même un liquide de refroidissement ou de l'eau, résultant par exemple d'une défaillance mécanique, la phase de l'impédance de l'échantillon d'huile augmente (voir notamment les figures 7 et 8 annexées).

Plus précisément encore, les essais conduits par la demanderesse ont révélé une pente de signe négatif avant la pollution puis de sens positif après celle-ci.

Ce phénomène est schématisé sur la figure 13 sur laquelle on a illustré d'une part, l'évolution de la phase de l'impédance ϕ et d'autre part, l'évolution de la pente de cette phase dϕ/dt.

Dans ce contexte, les moyens 10 conformes à la présente invention comprennent des moyens aptes à mesurer la pente de la phase de l'impédance et des moyens aptes à comparer les évolutions de cette pente avec un seuil pour générer une alarme "de type défaillance mécanique" lorsque la variation de pente de la phase de l'impédance dépasse un seuil déterminé. La génération de cette alarme "de type défaillance mécanique" incite l'usager à se diriger rapidement vers une station service, ce type d'alarme visualisant en principe une défaillance mécanique conduisant à une pollution grave de l'huile par un corps étranger tel que, comme indiqué précédemment, un carburant par exemple essence ou diesel, voir du fluide de refroidissement ou de l'eau.

Par ailleurs, les essais conduits par la demanderesse ont démontré que l'évolution de la phase de l'impédance de l'échantillon d'huile varie en fonction de la température (voir par exemple figures 9 à 11).

Pour cette raison, de préférence, les moyens 10 comprennent en outre un capteur de température et des moyens aptes à corriger la valeur de la phase de l'impédance utilisée pour le traitement, en fonction de la température mesurée, sur la base d'une abaque ou équivalent pré-établie.

## Revendications

1. Dispositif de mesure de l'usure d'un lubrifiant, par exemple d'huile sur véhicule automobile, comprenant un dispositif (10) de mesure de la phase de l'impédance du lubrifiant **caractérisé par le fait que** les moyens de mesure comprennent des moyens de mesure de la pente de la phase de l'impédance (dϕ/dt) et des moyens aptes à comparer l'évolution dans le temps de la pente de la phase de l'impédance (dϕ/dt) avec une valeur seuil et pour générer une alarme "de défaillance mécanique" si l'évolution de la pente de la phase de l'impédance (dϕ/dt) dépasse un seuil prédéterminé.

2. Dispositif selon la revendication 1, **caractérisé par le fait qu'**il comprend des moyens aptes à comparer l'amplitude de la phase de l'impédance du lubrifiant avec une valeur seuil (S1) et des moyens aptes à générer une alerte "de vidange ou maintenance" quand le seuil précité (S1) est franchi par défaut.

3. Dispositif selon la revendication 2, **caractérisé par le fait que** le seuil (S1) considéré est un seuil relatif basé sur la mesure de la valeur initiale de la phase de l'impédance.

4. Dispositif selon l'une des revendications 1 à 3, **caractérisé par le fait qu'**il comprend des moyens aptes à appliquer une tension alternative entre deux électrodes (20, 22) plongées dans un échantillon de lubrifiant à surveiller, et des moyens aptes à mesurer la phase du courant correspondant par rapport à ladite tension appliquée.

5. Dispositif selon la revendication 4, **caractérisé par le fait que** les électrodes (20, 22) sont formées d'électrodes cylindriques concentriques.

6. Dispositif selon l'une des revendications 1 à 5, **caractérisé par le fait qu'**il comprend des moyens de mesure de la température et des moyens aptes à appliquer une correction à la mesure de la phase d'impédance en fonction de la température détectée.

## Claims

1. Device for measuring the degradation of a lubricant, for example motor vehicle oil, comprising a device (10) for measuring the lubricant impedance phase, **characterized in that** the measurement means comprise means for measuring the slope of the impedance phase (dϕ/dt) and means capable of comparing the change over time of the slope of the impedance phase (dϕ/dt) with a threshold value and for generating a "mechanical failure" alarm if the change of the slope of the impedance phase (dϕ/dt) exceeds a predetermined threshold.

2. Device according to Claim 1, **characterized in that** it comprises means capable of comparing the amplitude of the lubricant impedance phase with a threshold value (S1) and means capable of generating an "oil change or service" alert when the abovementioned threshold (S1) is crossed by default.

3. Device according to Claim 2, **characterized in that** the threshold (S1) in question is a relative threshold based on the measurement of the initial value of the impedance phase.

4. Device according to one of Claims 1 to 3, **characterized in that** it comprises means capable of applying an alternating voltage between two electrodes (20, 22) immersed in a sample of lubricant to be monitored, and means capable of measuring the phase of the corresponding current with respect to the said applied voltage.

5. Device according to Claim 4, **characterized in that** the electrodes (20, 22) are formed by concentric cylindrical electrodes.

6. Device according to one of Claims 1 to 5, **characterized in that** it comprises temperature-measuring means and means capable of applying a correction to the impedance phase measurement as a function of the temperature detected.

## Patentansprüche

1. Vorrichtung zur Messung der Abnutzung eines Schmiermittels, zum Beispiel des Öls in einem Kraftfahrzeug, die eine Vorrichtung (10) zur Messung der Phase der Impedanz des Schmiermittels umfaßt, **dadurch gekennzeichnet, daß** die Mittel zur Messung Mittel zur Messung der Steigung der Phase der Impedanz (dϕ/dt) und Mittel umfassen, die geeignet sind, die zeitliche Entwicklung der Steigung der Phase der Impedanz (dϕ/dt) mit einem Schwellenwert zu vergleichen und einen Alarm "mechanische Störung" zu erzeugen, wenn die Entwicklung der Steigung der Phase der Impedanz (dϕ/dt) einen bestimmten Schwellenwert übersteigt.

2. Vorrichtung nach Anspruch 1, **dadurch gekennzeichnet, daß** sie Mittel umfaßt, die geeignet sind, die Amplitude der Phase der Impedanz des Schmiermittels mit einem Schwellenwert (S1) zu vergleichen, sowie Mittel, die geeignet sind, eine Warnung "Ölwechsel oder Wartung" zu erzeugen, wenn der genannte Schwellenwert (S1) durch einen Defekt überschritten wird.

3. Vorrichtung nach Anspruch 2, **dadurch gekennzeichnet, daß** der betrachtete Schwellenwert (S1) ein relativer Schwellenwert ist, der auf der Messung des Anfangswertes der Phase der Impedanz beruht.

4. Vorrichtung nach einem der Ansprüche 1 bis 3, **dadurch gekennzeichnet, daß** sie Mittel, die geeignet sind, eine Wechselspannung zwischen zwei Elektroden (20, 22) anzulegen, die in eine Probe des zu überwachenden Schmiermittels tauchen, und Mittel umfaßt, die geeignet sind, die Phase des entsprechenden Stroms in Bezug auf die genannte, angelegte Spannung zu messen.

5. Vorrichtung nach Anspruch 4, **dadurch gekennzeichnet, daß** die Elektroden (20, 22) aus zylindrischen, konzentrischen Elektroden gebildet sind.

6. Vorrichtung nach einem der Ansprüche 1 bis 5, **dadurch gekennzeichnet, daß** sie Mittel zur Messung der Temperatur und Mittel umfaßt, die geeignet sind, eine Korrektur an die Messung der Phase der Impedanz in Funktion der erfaßten Temperatur anzulegen.
